# EUROPÄISCHE PATENTSCHRIFT

(11) **EP 2 122 363 B1**
(45) Veröffentlichungstag und Bekanntmachung des Hinweises auf die Patenterteilung: **04.01.2017**
(21) Anmeldenummer: 08734336.4
(22) Anmeldetag: 03.03.2008
(51) Int. Cl.: G01N 33/74

(54) **DIAGNOSE UND RISIKOSTRATIFIZIERUNG VON HERZINSUFFIZIENZ MITTELS NATRIURETISCHEN PEPTIDEN FÜR NYHA I PATIENTEN**
DIAGNOSIS AND RISK STRATIFICATION OF CARDIAC INSUFFICIENCY BY MEANS OF NATRIURETIC PEPTIDES FOR NYHA I PATIENTS
DIAGNOSTIC ET STRATIFICATION DES RISQUES D'UNE INSUFFISANCE CARDIAQUE AU MOYEN DE PEPTIDES NATRIURÉTIQUES POUR DES PATIENTS NYHA I

(30) Priorität: 03.03.2007 DE 102007010834
(43) Veröffentlichungstag der Anmeldung: 25.11.2009
(60) Teilanmeldung: 16189548.7
(73) Patentinhaber: B.R.A.H.M.S GmbH, 16761 Hennigsdorf (DE)
(72) Erfinder: BERGMANN, Andreas, 12351 Berlin (DE); MORGENTHALER, Nils, 13503 Berlin (DE); PAPASSOTIRIOU, Jana, 14163 Berlin (DE); STRUCK, Joachim, 13465 Berlin (DE); ANKER, Stefan, 13053 Berlin (DE)
(74) Vertreter: Simandi, Claus
(86) Internationale Anmeldenummer: PCT/DE2008/000357
(87) Internationale Veröffentlichungsnummer: WO 2008/106938

(56) Entgegenhaltungen:
- EP-A- 1 577 673
- EP-A- 1 901 073
- WO-A-2004/046181
- WO-A-2005/124364
- WO-A-2006/087373
- US-A- 5 498 524
- VON HAEHLING ET AL: "Comparison of Midregional Pro-Atrial Natriuretic Peptide With N-Terminal Pro-B-Type Natriuretic Peptide in Predicting Survival in Patients With Chronic Heart Failure" JOURNAL OF THE AMERICAN COLLEGE OF CARDIOLOGY, ELSEVIER, NEW YORK, NY, US, Bd. 50, Nr. 20, 29. Oktober 2007 (2007-10-29), Seiten 1973-1980, XP022332007 ISSN: 0735-1097

## Beschreibung

Die Erfindung betrifft ein Verfahren zur Diagnose und / oder Risikostratifizierung und / oder Outcome-Prognose von Herzinsuffizienz für NYHA I Patienten, wobei eine Bestimmung des Markers proANP oder Fragmente oder Teilpeptide davon, insbesondere NT-proANP oder Fragmente oder Teilpeptide davon, parallel zu einer Bestimmung von BNP, proBNP und /oder NT-proBNP an zu untersuchenden Patienten durchgeführt wird.

In Europa kommen jährlich etwa eine Million Patienten mit dem Symptom der akuten Atemnot in die Notaufnahmen von Kliniken. Die Atemnot ist ein Leitsymptom vieler Erkrankungen und lässt sich in ca. 35-47% der Fälle auf eine Herzinsuffizienz zurückführen (Januzzi JL Jr, Camargo CA, Anwaruddin S, Baggish AL, Chen AA, Krauser DG, Tung R, Cameron R, Nagurney JT, Chae CU, Lloyd-Jones DM, Brown DF, Foran-Melanson S, Sluss PM, Lee-Lewandrowski E, Lewandrowski KB, The N-terminal Pro-BNP investigation of dyspnea in the emergency department (PRIDE) study, Am J Cardiol. 95(8) (2005), pp. 948-954 und Maisel AS, Krishnaswamy P, Nowak RM, McCord J, Hollander JE, Duc P, Omland T, Storrow AB, Abraham WT, Wu AH, Clopton P, Steg PG, Westheim A, Knudsen CW, Perez A, Kazanegra R, Herrmann HC, McCullough PA; Breathing Not Properly Multinational Study Investigators, Rapid measurement of B-type natriuretic peptide in the emergency diagnosis of heart failure, N Engl J Med. 347(3) (2002), pp. 161-167).

Im Anfangsstadium bemerkt der Patient oft nur wenig von der Herzinsuffizienz. Unbehandelt nimmt die Erkrankung in der Regel an Schwere zu und führt im Spätstadium zu völliger körperlicher Erschöpfung bereits in Ruhe. Die Unterversorgung aller Körperorgane, einschließlich des Herzmuskels selbst, kann in diesem Stadium zum Tode führen. Ist die Erkrankung erst einmal fortgeschritten, ist die Lebenserwartung auch unter optimaler Therapie stark vermindert (ca. 30% Todesfälle pro Jahr). Es ist daher wichtig, eine Herzschwäche möglichst früh zu erkennen und ihre Ursachen konsequent anzugehen.

Um mit einer geeigneten Therapie zu beginnen, bedarf es daher einer frühen Diagnose und Differenzierung der zugrunde liegenden Erkrankung bereits im Frühstadium und in der Not- und Intensivmedizin. Aufgrund unspezifischer Symptome (Luftnot, Husten) ist sowohl die Differenzierung und Abgrenzung der Herzinsuffizienz von anderen Erkrankungen häufig erschwert.

Mittels Bestimmung der Plasmakonzentration des brain natriuretic peptide (BNP bzw. NTproBNP) steht ein Test zur Verfügung, der auch in der Alltagsroutine für die Diagnostik einer Herzinsuffizienz erfolgreich eingesetzt wird (Maisel et al. (supra)). NT-proBNP wird im Stand der Technik zur Verlaufskontrolle von Herzinsuffizienz eingesetzt.

Im Stand der Technik ist ebenfalls bereits proANP als Marker beschrieben. US5498524 beschreibt den Einsatz von proANP zur Diagnose von Herzinsuffizienz bei asymptomatischen Patienten. EP721105B1 beschreibt ein Verfahren zur Bestimmung von proANP mittels geeigneten Antikörpern für Herzerkrankungen.

WO 2006/087373 beschreibt die Verwendung von pro ANP und pro BNP zur Diagnose von Herzterankneiten insbesondere Herzvosagen.

Es besteht jedoch im Stand der Technik ein hohes Bedürfnis für Patienten mit der Indikation Herzinsuffizienz insbesondere die Diagnose, Risikostratifizierung und outcome-Prognose zu verbessern.

Daher ist es Aufgabe der vorliegenden Erfindung ein Verfahren zur verbesserten Diagnose und / oder Risikostratifizierung und / oder outcome-Prognose von Herzinsuffizienz bereitzustellen.

Nachteilig an bekannten Diagnoseverfahren unter Verwendung der bisher bekannten Markern ist jedoch, dass eine frühzeitige und vollständige Erfassung von Risikopatienten nicht vollständig gelingt und daher eine Risikostratifizierung nur ungenügend erfolgt. Eine der Erfindung weitere zugrunde liegende Aufgabe besteht daher darin, ein Verfahren zur Risikostratifizierung von Herzinsuffizienz zu entwickeln, das eine verbesserte Erfassung von Risikopatienten ermöglicht, dies vor allem für Subgruppen von Patienten.

Überraschender Weise konnte nunmehr gezeigt werden, dass bei der Bestimmung von proANP oder Fragmente oder Teilpeptide davon, insbesondere NT-proANP oder Fragmente oder Teilpeptide davon in paralleler Bestimmung von proBNP, NT-proBNP und / oder BNP eine Verbesserung der Diagnose, Risikostratifizierung und Outcome-Prognose von Herzinsuffizienz und zwar bei einer symptomatischen Herzinsuffizienz ohne Beschwerden (NYHA-Stadium I), insbesondere Linksherzinsuffizienz ohne Beschwerden (NYHA-Stadium I), erreicht werden kann.

Die Aufgabe wird daher durch ein Verfahren nach Anspruch 1 gelöst (nachstehend erfindungsgemäßes Verfahren).

Daher erlaubt das erfindungsgemäße Verfahren besonders vorteilhaft die diagnostische und prognostische Wertigkeit für Patienten der Klasse NYHA I zu verbessern.

In einer besonders bevorzugten Ausführungsform der Erfindung weisen die Patienten zudem einen Body Mass-index (BMI) von mindestens 30 kg/m² auf. Dies ist ein zusätzlicher Parameter der zusätzlich signifikant die diagnostische und prognostische Wertigkeit verbessert.

Im Rahmen dieser Erfindung wird unter "Herzinsuffizienz" ein akutes oder chronisches Unvermögen des Herzens, die Gewebe mit ausreichend Blut und infolge dessen genügend Sauerstoff zu versorgen, um den Gewebestoffwechsel in Ruhe oder unter Belastung sicherzustellen. Klinisch liegt eine Herzinsuffizienz vor, wenn typische Symptome (Dyspnoe, Müdigkeit, Flüssigkeitsretention) bestehen, deren ursächlich eine kardiale Funktionsstörung im Sinne einer systolischen oder diastolischen Funktionsstörung zugrunde liegt. Ebenfalls die chronische Herzinsuffizienz (CHF) ist erfindungsgemäß umfasst(Kardiologie compact, herausgegeben von Christian Mewis, Reimer Riessen und Ioakim Spyridopoulos, 2. unveränderte Auflage, Thieme 2006). Ursachen einer Herzinsuffizienz können sein: Herzklappenfehler (z. B. als Spätfolge des rheumatischen Fiebers), Myokarditis (Herzmuskelentzündung), Herzrhythmusstörungen, Herzinfarkt neben zu hohem Blutdruck (Hypertonie) und/oder Arteriosklerose (Verkalkung) der Herzkranzgefäße (koronare Herzkrankheit). Weiterhin erfindungsgemäß umfasst sind hypertensive Herzkrankheit mit (kongestiver) Herzinsuffizienz, Hypertensive Herz- und Nierenkrankheit mit (kongestiver) Herzinsuffizienz. Die vorliegende Erfindung betrifft solche Patienten die eine Herzinsuffizienz ohne Beschwerden (NYHA-Stadium I) aufweisen. (NYHA = Klassifikation der New York Heart Association (Hoppe UC et al.: Leitlinien zur Therapie der chronischen Herzinsuffizienz. Z Kardiol (2005) 94:488-509)).

Der Begriff "Risikostratifizierung" umfasst erfindungsgemäß das Auffinden von Patienten, insbesondere Notfallpatienten und Risikopatienten, mit der schlechteren Prognose, zwecks intensiverer Diagnostik und Therapie/Behandlung von Herzinsuffizienz mit dem Ziel eines möglichst günstigen Verlaufs zu ermöglichen. Eine erfindungsgemäße Risikostratifizierung erlaubt in Folge ein effektives Behandlungsverfahren, die betreffend einer Herzinsuffizienz ohne Beschwerden (NYHA-Stadium I) gegeben sind.

Besonders vorteilhaft kann insbesondere in Fällen der Notfall- und /oder Intensivmedizin mittels des erfindungsgemäßen Verfahrens eine sichere Diagnose oder Outcome-Prognose erfolgen. Das erfindungsgemäße Verfahren ermöglicht klinische Entscheidungen, die zu einem schnellen Therapieerfolg führen. Solche klinischen Entscheidungen umfassen ebenfalls weiterführende Behandlungen mittels Arzneimitteln zur Behandlung oder Therapie von Herzinsuffizienz, solche wie ACE-Hemmer, AT1-Antagonisten: Blocker des Angiotensin-II-Rezeptors (Subtyp 1), Betablocker Bisoprolol, Carvedilol, Metoprolol und Nebivolol, Vasopressin-Rezeptor-Antagonisten, Aldosteronantagonisten ab NYHA-Stadium III, Kalzium-Sensitizer (Levosimendan).

In einer weiteren bevorzugten Ausführungsform betrifft das erfindungsgemäße Verfahren daher die Therapiesteuerung einer Herzinsuffizienz ohne Beschwerden (NYHA-Stadium I).

Daher betrifft die Erfindung ebenfalls ein Verfahren zur Risikostratifizierung von Patienten, insbesondere zur Stratifizierung von Patienten für klinische Entscheidungen, vorzugsweise in der zeitlich kritischen Intensivmedizin oder Notfallmedizin und zur Hospitalisierung von Patienten.

In einer weiteren bevorzugten Ausführungsform des erfindungsgemäßen Verfahrens erfolgt die Diagnose zur Prognose, vorzugsweise zur Outcome-Prognose, zur differentialdiagnostischen Früherkennung und Erkennung, zur Beurteilung des Schweregrades und zur therapiebegleitenden Verlaufsbeurteilung für Patienten mit einer Herzinsuffizienz ohne Beschwerden (NYHA-Stadium I).

In einer weiteren Ausführungsform des erfindungsgemäßen Verfahrens wird dem zu untersuchenden Patienten Körperflüssigkeit, insbesondere Blut entnommen, wahlweise Vollblut oder Serum, und die Diagnose erfolgt *in vitro*/*ex vivo,* d.h. außerhalb des menschlichen oder tierischen Körpers. Aufgrund der Bestimmung des Markers proANP oder Fragmente oder Teilpeptide davon, insbesondere NT-proANP oder Fragmente oder Teilpeptide davon und seiner vorhandenen Menge in mindestens einer Patientenprobe kann die Diagnose erfolgen. Parallel wird erfindungsgemäß eine Bestimmung des Markers proBNP, NT-proBNP und / oder BNP in seiner vorhandenen Menge in mindestens einer Patientenprobe vorgenommen.

Im Rahmen dieser Erfindung wird unter "proANP" (auch: NT-proANP) ein freies 98 Aminosäuren enthaltendes Polypeptid/Protein eines atrialen natriuretischen Peptids verstanden. Das N-terminale Fragment proANP (AS 1-98) entsteht aus der Abspaltung des zirkulierenden Hormons alpha-ANP (99-126 mit 28AS) aus dem Prohormon "proANP" (AS 1-126, siehe SEQ ID No. 1), welches aus 126 Aminosäuren besteht und in den Sekretgranula der myoendokrinen Zellen gespeichert wird und erfindungsgemäß umfasst ist. Ferner kann dieses erfindungsgemäße Polypeptid posttranslationale Modifikationen aufweisen, wie Glykolisierung, Lip(o)idisierung oder Derivatisierungen. Insbesondere NT-proANP (AS 1-98) ist überaus stabil im Plasma.

Fragmente und Teilpeptide insbesondere der midregionalen Bereich der AS 50-90 von NT-proANP (1-98) bzw. proANP (1-126) sind bekannt. Erfindungsgemäß ist insbesondere das Fragment mit den AS 53-90 des NT-proANP (1-98) bevorzugt (auch: MR-proANP genannt, siehe WO2004046181 und SEQ ID No. 1). Ein geeigneter Assay ist in WO2004046181A1 (BRAHMS AG) offenbart. Weiterhin bevorzugt ist NT-proANP (AS 1-98). Das Fragment (alpha)-ANP (AS 99-126) ist ebenfalls beschrieben.

Im Rahmen dieser Erfindung wird unter BNP (AS 77-108) ein B-Typ Natriuretisches Peptid verstanden, welches aus dem Prohormon proBNP (AS 1-108, siehe SEQ ID No. 2) abgespalten wird, wobei zugleich das NT-proBNP (AS 1-76) entsteht. NT-proBNP ist erfindungsgemäß bevorzugt.

"Parallele Bestimmung" bedeutet, dass die Bestimmungen insbesondere simultan oder gleichzeitig erfolgen oder zumindest eine ausreichende Zuordnung oder kombinierte Auswertung erlauben.

In einer weiteren Ausführungsform kann die Bestimmung von proANP, NT-proANP oder jeweils Fragmente oder Teilpeptide davon parallel zu der Bestimmung von BNP, proBNP und / oder NT-proBNP zusätzlich mit weiteren Markern erfolgen und zwar vorzugsweise solche, die bereits auf eine Herzinsuffizienz hinweisen und einen synergetischen Effekt von Markerkombinationen im erfindungsgemäßen Verfahren erlauben.

Daher betrifft die Erfindung eine solche Ausführungsform des erfindungsgemäßen Verfahrens, wobei die Bestimmung zusätzlich mit mindestens einem weiteren Marker ausgewählt aus der Gruppe inflammatorischer Marker, cardiovaskulärer Marker oder ischämischer Marker an einem zu untersuchenden Patienten durchgeführt wird.

Erfindungsgemäß kann der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt sein sowie der cardiovaskuläre Marker aus mindestens einem Marker aus der Gruppe Kreatinkinase, Myeloperoxidase, Copeptin, Myoglobin, kardiale Troponin, CRP ausgewählt sein. Ferner werden hierunter ebenfalls Kreislaufregulierende (Pro)Hormone verstanden, insbesondere wie pro-Gastrin-Releasing Peptid (proGRP), pro-Endothelin (proEnd), pro-Leptin, pro-Neuropeptid-Y, pro-Somatostatin, pro- Neuropeptid-YY, pro-Opiomelanocortin, pro-Adrenomedullin (proADM), Copeptin oder jeweils eine Teilsequenz davon.

Der ischämische Marker kann aus mindestens einem Marker aus der Gruppe Troponin I und T, CK-MB ausgewählt sein.

In einer weiteren Ausführungsform der Erfindung kann das erfindungsgemäße Verfahren mittels parallelen oder simultanen Bestimmungen der Marker durchgeführt werden (z.B. Multititerplatten mit 96 und mehr Kavitäten), wobei die Bestimmungen an mindestens einer Patientenprobe durchgeführt werden.

Ferner kann das erfindungsgemäße Verfahren und dessen Bestimmungen an einem Analyseautomaten, insbesondere mittels einem Kryptor (http://www.kryptor.net/) durchgeführt werden.

In einer weiteren Ausführungsform kann das erfindungsgemäße Verfahren und dessen Bestimmungen mittels einem Schnelltest durchgeführt werden (z.B. lateral-flow Test), vorzugsweise in Multiparameterbestimmung.

Ein weiterer Erfindungsgegenstand betrifft die Verwendung der Marker proANP oder NT-proANP parallel zu einer Bestimmung der Marker proBNP, NT-proBNP und / oder BNP an einem zu untersuchenden Patienten zur in-vitro Diagnose und / oder Risikostratifizierung und / oder zur Outcome-Prognose von Herzinsuffizienz ohne Beschwerden (NYHA-Stadium I). MR-proANP ist ein bevorzugtes Fragment oder Teilpeptid. Ebenfalls bezieht sich die erfindungsgemäße Verwendung auf weitere oben genannte Ausführungsformen.

Nachfolgende Beispiele und Figuren dienen zur näheren Erläuterung der Erfindung, jedoch ohne die Erfindung auf diese Beispiele und Figuren zu beschränken.

### Beispiele und Figuren:

Beispiel 1: Patienten in NHYA-Klasse 1: Receiver Operator Characteristic (ROC, StatView 5.0 Software für Windows (Abacus, Concepts, Berkley, Canada und MedCalc, Broekstraat, Mariakerke, Belgien)-Analysen für Überleben/Versterben MR-proANP wurde aus dem Plasma der Patienten isoliert und bei -80 Grad Celsius gefroren. Zum Nachweis von MR-proANP wurde ein Sandwich-Assay (LIA) gemäß WO2004046181 der BRAHMS AG verwendet (Morgenthaler et al. Immunoluminometric assay fort he midregion of pro-atrial natriuretic peptide in human plasma, Clin. Chem, 2004; 50: 234-236).

NT-proBNP wurde mit ELICIA (Roche Diagnostics, Penzberg, Deutschland) bestimmt.

Probandenzahl n=66 / 7 Patienten davon sind verstorben.

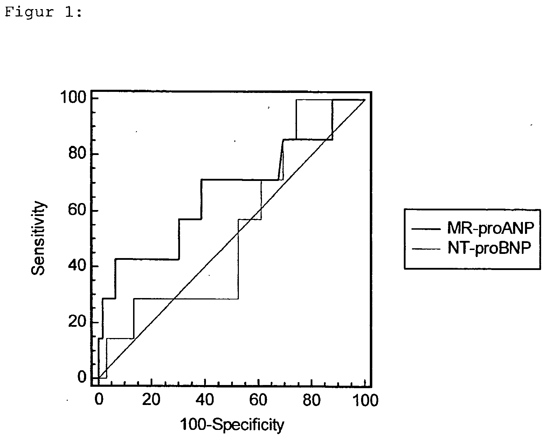

Aus Figur 1 ergibt sich eine Area under the curve (AUC) für MR-proANP = 0,665 und für NT-proBNP = 0,533.
MR-proANP: 0,665 - 0,5 (nicht signifikant): 0,165
NT-proBNP: 0,533 - 0,5 (nicht signifikant): 0,033
0,165/0,033 = 5

Demnach ergibt sich eine 5-fach gesteigerte diagnostische Wertigkeit (Betrachtung Überleben/Versterben) für MR-proANP im Vergleich zu NT-proBNP in der Subgruppe von Patienten in der NHYA-Klasse 1.

### Beispiel 2:

Bestätigung der gesteigerten prognostischen Wertigkeit von MR-proANP im Vergleich zu NT-proBNP mittels Cox-Regression

### Univariate Analyse:

Vorgehen: In der Subgruppe der NYHA-1 Patienten wurde für beide Marker (MR-proANP und NT-proBNP) der Median berechnet; für NT-proBNP ergab sich dabei ein 3,7 fach höherer Median als für MR-proANP. Daher wurde in der Cox-Analyse die RiskRatio für NT-proBNP im Vergleich zu MR-proANP für einen 3,7 fach großen Konzentrations-Anstieg berechnet.

### Ergebnis der univariaten Cox Proportional Hazard Analyse:

| Marker | ChiSquare | RiskRatio (95% CI) | p |
|---|---|---|---|
| MR-proANP | | | |
| (pro 100 pmol/L Anstieg) | 6.758 | 2.517 (1.255-5.047) | 0.009 |
| NT-proBNP | | | |
| (370 pg/mL Anstieg) | 0.383 | 1.107 (0.803-1.525) | 0.536 |

Unabhängig davon, daß sich für NT-proBNP kein signifikantes Ergebnis ergab (p=0,536), verdeutlicht die Risk Ratio von 2.517 für MR-proANP im Vergleich zu NT-proBNP (1.107) eine Verbesserung in der Subgruppe der NYHA-1 Patienten. Eine RiskRatio von 1.000 zeigt an, daß das Risiko zu Versterben pro Konzentrationseinheit nicht ansteigt. Für MR-proANP bedeutet eine RiskRatio von 2.517, daß das Risiko zu Versterben pro Einheit (hier 100 pmoL/L) um 151,7% ansteigt. Im Vergleich zu NT-proBNP ist das ein 14,2 fach höherer Anstieg des Risikos.

### Multivariates Modell:

| Marker | ChiSquare | RiskRatio (95% CI) | | p |
|---|---|---|---|---|
| LN MR-proANP | 5,861 | 25,554 | (1,854-352,272) | 0,015 |
| LN NT-proBNP | 2,093 | 0,429 | (0,136-1,350) | 0,148 |
| Alter | 1,673 | 0,943 | (0,863-1,031) | 0,196 |

Das Ergebnis verdeutlicht wieder die Verbesserung im Einsatz von MR-proANP, weil es in einem Modell mit NT-proBNP und Alter als einziger unabhängiger Prädiktor für Versterben in der NYHA-1 Subgruppe bestehen bleibt (p=0,015).

### Beispiel 3: Patienten mit einem BMI >=30 kg/m²

Darlegung der Steigerung der prognostischen Wertigkeit von MR-proANP im Vergleich zu NT-proBNP mittels Cox-Regression (Probandenzahl: n=114; 36 Patienten davon sind verstorben)

### Univariate Analyse:

Vorgehen: In der Subgruppe der Patienten mit einem BMI >= 30 kg/m² wurde für beide Marker (MR-proANP und NT-proBNP) der Median berechnet; für NT-proBNP ergab sich dabei ein 5 fach höherer Median als für MR-proANP. Daher wurde in der Cox-Analyse die RiskRatio für NT-proBNP im Vergleich zu MR-proANP für einen 5fach so großen Konzentrations-Anstieg berechnet.

### Ergebnis der univariaten Cox Proportional Hazard Analyse:

| Marker | ChiSquare | RiskRatio (95% CI) | p |
|---|---|---|---|
| MR-proANP | | | |
| (100 pmol/L Anstieg) | 14.414 | 1.503 (1.217-1.855) | <0.0001 |
| NT-proBNP | | | |
| (500 pg/mL Anstieg) | 0.662 | 1.024 (0.963-1.094) | 0.416 |

Unabhängig davon, daß sich für NT-proBNP kein signifikantes Ergebnis ergab (p=0,416), verdeutlicht die Risk Ratio von 1.503 für MR-proANP im Vergleich zu NT-proBNP (1.024) Überlegenheit in der Subgruppe der Patienten mit einem BMI >=30. Eine RiskRatio von 1.000 zeigt an, daß das Risiko zu Versterben pro Konzentrationseinheit nicht ansteigt. Für MR-proANP bedeutet eine RiskRatio von 1.503 demnach, daß das Risiko zu Versterben pro Einheit (hier 100 pmoL/L) um 50,3% ansteigt. Im Vergleich zu NT-proBNP ist das ein 21 fach höherer Anstieg des Risikos.

### Multivariates Modell:

| Marker | ChiSquare | RiskRatio (95% CI) | p |
|---|---|---|---|
| LN MR-proANP | 7.198 | 2.776 (1.317-5.854) | 0.007 |
| LN NT-proBNP | 0.005 | 0.985 (0.644-1.506) | 0.943 |

Das Ergebnis verdeutlicht wieder die prognostische Steigerung des MR-proANP, weil es in einem Modell mit NT-proBNP als unabhängiger Prädiktor für Versterben in der Subgruppe der Patienten mit einem BMI >=30 bestehen bleibt (p=0,007). NT-proBNP ist dagegen kein unabhängiger Prädiktor.

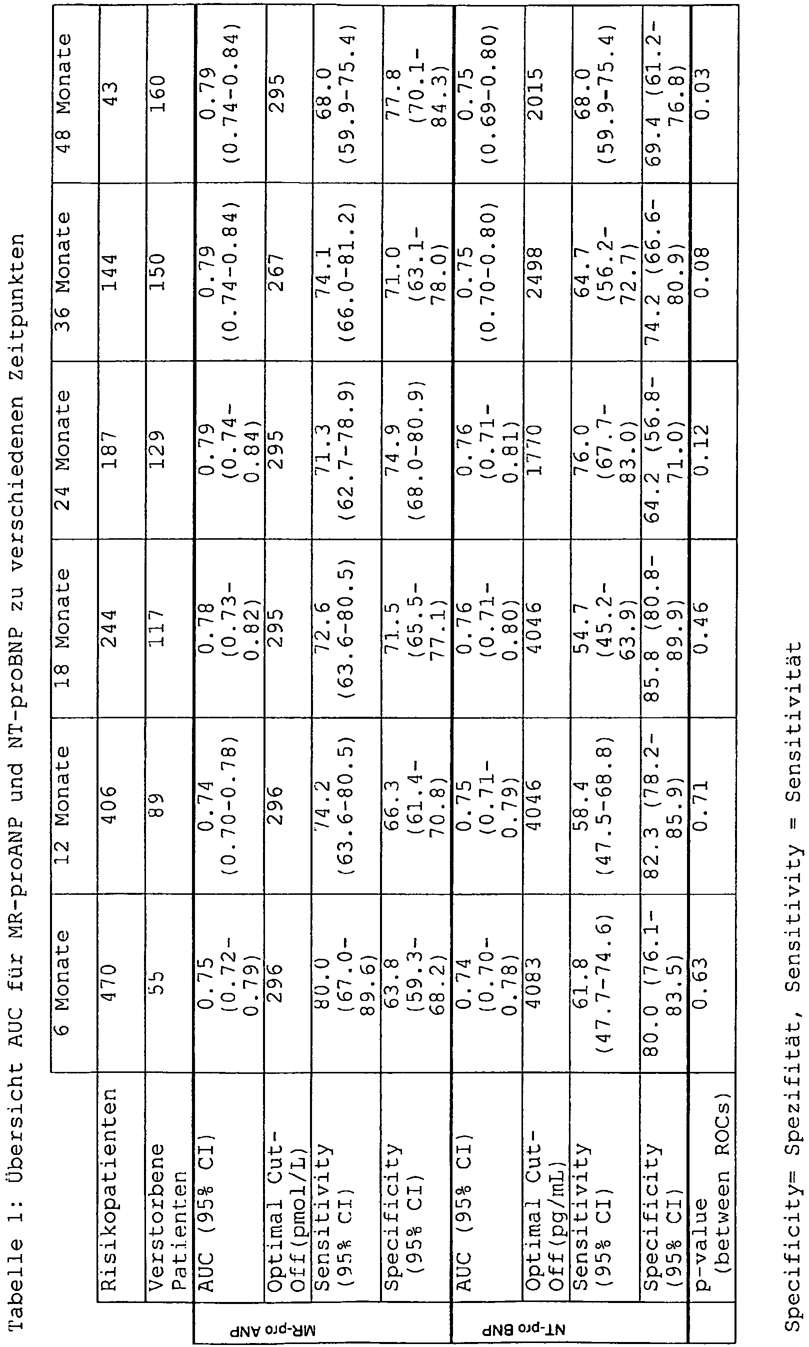

**Tabelle 2: Cox Proportional Hazard Analyse für MT-proANP und NT-proBNP zur Vorhersage der Überlebenden in verschiedenen Stadien der Herzinsuffizienz**

| | NT-proBNP (per 1000pg/mL increase) (n=774) | | | MR-proANP (per 100pmol/L increase) (n=774) | | | | |
|---|---|---|---|---|---|---|---|---|
| Disease Severity | Chi Square | RR (95%CI) | P | Chi Square | RR (95%CI)) | P | Joint Chi Square | Added Prognostic Power * |
| NYHA I (mild) | 0.38 | 1.32 (0.55-3.13) | 0.54 | 6.76 | 2.52 (1.26-5.05) | 0.0093 | 7.16 | 1679% |
| NYHA II&III (moderate) | 70.5 | 1.11 (1.08-1.14) | 0.0001 | 105.0 | 1.19 (1.15-1.23) | 0.0001 | 106.6 | 49% |
| NYHA IV (severe) | 5.14 | 1.03 (1.00-1.05) | 0.023 | 6.65 | 1.09 (1.02-1.15) | 0.0099 | 7.77 | 29% |

| | | | | | | | | |
|---|---|---|---|---|---|---|---|---|
| *= Zusätzliche prognostische Wertigkeit für MR-proANP Probandenzahl n=774 Disease Severity = Schweregrad der Erkrankung, RR = RiskRatio, increase = Zunahme | | | | | | | | |

**Tabelle 3: Klassifikation der New York Heart Association (NYHA)**

| | |
|---|---|
| NYHA I | Keine körperliche Einschränkung. Alltägliche körperliche Belastung verursacht keine inadäquate Erschöpfung, Rhythmusstörungen, Luftnot oder Angina Pectoris. |
| NYHA II | Leichte Einschränkung der körperlichen Belastbarkeit. Keine Beschwerden in Ruhe. Erschöpfung, Rhythmusstörungen, Luftnot oder Angina pectoris bei alltäglicher körperlicher Belastung. |
| NYHA III | Höhergradige Einschränkung der körperlichen Leistungsfähigkeit bei gewohnter Tätigkeit. Keine Beschwerden in Ruhe. Erschöpfung, Rhythmusstörungen, Luftnot oder Angina pectoris bei geringer körperlicher Belastung. |
| NYHA IV | Beschwerden bei allen körperlichen Aktivitäten und in Ruhe. Bettlägerigkeit. |

## Patentansprüche

1. Verfahren zur in-vitro Diagnose und / oder Risikostratifizierung und / oder zur Outcome-Prognose von Herzinsuffizienz ohne Beschwerden im NYHA-Stadium I,
**dadurch gekennzeichnet,**
**dass** eine Bestimmung des Markers proANP oder NT-proANP oder MR-proANP (AS 53-90 des NT-proANP) parallel zu einer Bestimmung des Markers proBNP, NT-proBNP und / oder BNP von einem zu untersuchenden Patienten durchgeführt wird.

2. Verfahren zur in-vitro Diagnose und / oder Risikostratifizierung und / oder zur Outcome-Prognose von Herzinsuffizienz ohne Beschwerden im NYHA-Stadium I nach Anspruch 1, **dadurch gekennzeichnet, dass** der Marker MR-proANP (AS 53-90 des NT-proANP) ist.

3. Verfahren zur in-vitro Diagnose und / oder Risikostratifizierung und / oder zur Outcome-Prognose von Herzinsuffizienz ohne Beschwerden im NYHA-Stadium I nach Anspruch 1 oder 2, **dadurch gekennzeichnet, dass** der Patient einen Body Mass-index von mindestens 30 kg/m² aufweist.

4. Verfahren zur in-vitro Diagnose und / oder Risikostratifizierung und / oder zur Outcome-Prognose von Herzinsuffizienz ohne Beschwerden im NYHA-Stadium I nach einem der Ansprüche 1 bis 3 zum Ermöglichen von klinischen Entscheidungen, insbesondere weiterführende Behandlung und Therapie mittels Arzneimitteln, insbesondere in der Intensivmedizin oder Notfallmedizin und zur Hospitalisierung des Patienten.

5. Verfahren zur in-vitro Diagnose und / oder Risikostratifizierung und / oder zur Outcome-Prognose von Herzinsuffizienz ohne Beschwerden im NYHA-Stadium I nach einem der Ansprüche 1 bis 4, zur differentialdiagnostischen Früherkennung und Erkennung, zur Beurteilung des Schweregrades und zur therapiebegleitenden Verlaufsbeurteilung.

6. Verfahren nach einem der vorherigen-Ansprüche,
**dadurch gekennzeichnet,**
**dass** zusätzlich eine Bestimmung mindestens eines weiteren Marker ausgewählt aus der Gruppe inflammatorischer Marker, cardiovaskulärer Marker oder ischämischer Marker an einem zu untersuchenden Patienten durchgeführt wird.

7. Verfahren nach einem der vorherigen Ansprüche, **dadurch gekennzeichnet, dass** der inflammatorische Marker aus mindestens einem Marker aus der Gruppe C-reaktivem Protein (CRP), Cytokinen, wie etwa TNF-alpha, Interleukinen, wie etwa IL-6, Procalcitonin (1-116, 3-116) und Adhäsionsmolekülen, wie VCAM oder ICAM ausgewählt ist.

8. Verfahren nach einem der vorherigen Ansprüche, **dadurch gekennzeichnet, dass** der cardiovaskuläre Marker aus mindestens einem Marker aus der Gruppe Kreatinkinase, Myeloperoxidase, Copeptin, Myoglobin, kardiale Troponin, CRP sowie Kreislaufregulierende (Pro)Hormone, wie pro-Gastrin-Releasing Peptid (proGRP), pro-Endothelin-1, pro-Leptin, pro-Neuropeptid-Y, pro-Somatostatin, pro-Neuropeptid-YY, pro-Opiomelanocortin, pro-Adrenomedullin (proADM), Copeptin oder jeweils eine Teilsequenz davon ausgewählt ist.

9. Verfahren nach einem der vorherigen Ansprüche, **dadurch gekennzeichnet, dass** der ischämischer Marker aus mindestens einem Marker aus der Gruppe Troponin I und T, CK-MB ausgewählt ist.

10. Verfahren nach einem der vorherigen Ansprüche,
**dadurch gekennzeichnet,**
**dass** parallele oder simultane Bestimmungen der Marker durchgeführt werden.

11. Verfahren nach einem der vorherigen Ansprüche,
**dadurch gekennzeichnet,**
**dass** die Bestimmungen an mindestens einer Patientenprobe durchgeführt werden.

12. Verfahren nach einem der vorherigen Ansprüche,
**dadurch gekennzeichnet,**
**dass** die Bestimmungen an einem Analyseautomaten, insbesondere mittels einem Kryptor, durchgeführt werden.

13. Verfahren nach einem der vorherigen Ansprüche,
**dadurch gekennzeichnet, dass** die Bestimmungen mittels einem Schnelltest, insbesondere in Einzel- oder Multiparameterbestimmungen durchgeführt werden.

14. Verwendung der Marker proANP oder NT-proANP oder MR-proANP (AS 53-90 des NT-proANP) parallel zu einer Bestimmung der Marker proBNP, NT-proBNP und / oder BNP von einem zu untersuchenden Patienten zur in-vitro Diagnose und / oder Risikostratifizierung und / oder zur Outcome-Prognose von Herzinsuffizienz ohne Beschwerden im NYHA-Stadium I.

## Claims

1. A method for the in-vitro diagnosis, and/or risk stratification, and/or outcome prognosis of cardiac insufficiency without any discomforts in NYHA stage I,
**characterized in that**
a determination of the marker proANP or NT-proANP, or MR-proANP (AS 53-90 of NT-proANP) is carried out parallel to a determination of the marker proBNP, NT-proBNP, and/or BNP from a patient who is to be examined.

2. The method for the in-vitro diagnosis, and/or risk stratification, and/or outcome prognosis of cardiac insufficiency without any discomforts in NYHA stage I according to claim 1, **characterized in that** the marker is MR-proANP (AS 53-90 of NT-proANP).

3. The method for the in-vitro diagnosis, and/or risk stratification, and/or outcome prognosis of cardiac insufficiency without any discomforts in NYHA stage I according to claims 1 or 2, **characterized in that** the patient has a body mass index of at least 30 kg/m².

4. The method for the in-vitro diagnosis, and/or risk stratification, and/or outcome prognosis of cardiac insufficiency without any discomforts in NYHA stage I according to one of the claims 1 to 3, for enabling clinical decisions, particularly the continuative treatment and therapy by means of medication, particularly in intensive medicine or emergency medicine and for the hospitalization of patients.

5. The method for the in-vitro diagnosis, and/or risk stratification, and/or outcome prognosis of cardiac insufficiency without any discomforts in NYHA stage I according to one of the claims 1 to 4, for the differential diagnostic early detection and detection, assessment of severity, and for therapy accompanying assessment of course.

6. The method according to one of the previous claims, **characterized in that** a determination of at least one further marker, selected from the group of inflammatory markers, cardiovascular markers, or ischemic markers, is additionally carried out on a patient to be examined.

7. The method according to one of the previous claims, **characterized in that** the inflammatory marker is selected from at least one marker from the group of C-reactive protein (CRP), ctyokins, such as TNF-alpha, interleukins, such as IL-6, procalcitonin (1-116, 3-116), and adhesion molecules, such as VCAM or ICAM.

8. The method according to one of the previous claims, **characterized in that** the cardiovascular marker is selected from at least one marker from the group of creatine kinesis, myeloperoxidasis, copeptin, myoglobin, cardiac troponin, CRP, and circulation regulating (pro)hormones, such as pro-gastrin releasing peptide (proGRP), pro-endothelin (proEnd), pro-leptin, pro-neuropeptide-Y, pro-somatostatin, pro-neuropeptide-YY, pro-opiomelanocortin, pro-adrenomedullin (proADM), copeptine, or a partial sequence thereof.

9. The method according to one of the previous claims, **characterized in that** the ischemic marker is selected from at least one marker from the group of troponin I and T, CK-MB.

10. The method according to one of the previous claims, **characterized in that** parallel or simultaneous determinations of the markers are carried out.

11. The method according to one of the previous claims, **characterized in that** the determinations are carried out on at least one patient sample.

12. The method according to one of the previous claims, **characterized in that** the determinations are carried out on an automatic analyzer, particularly by means of a Kryptor.

13. The method according to one of the previous claims, **characterized in that** the determinations are carried out by means of a rapid test, particularly in individual or multi-parameter determinations.

14. A use of the markers proANP or NT-proANP, or MR-proANP (AS 53-90 of NT-proANP) parallel to a determination of the markers proBNP, NT-proBNP, and/or BNP from a patient who is to be examined for the in-vitro diagnosis, and/or risk stratification, and/or outcome prognosis of cardiac insufficiency without any discomforts in NYHA stage I.

## Revendications

1. Procédé pour le diagnostic in vitro, et/ou la stratification du risque, et/ou le résultat de pronostic d'une insuffisance cardiaque sans aucun malaise chez des patients de la classe I de NYHA, **caractérisé en ce**
**qu'**une détermination du marqueur proANP, ou NT-proANP, ou MR-proANP (AS 53-90 du NT-proANP) est réalisée parallèlement à une détermination du marqueur proBNP, NT-proBNP, et/ou BNP d'un patient qui doit être examiné.

2. Procédé pour le diagnostic in vitro, et/ou la stratification du risque, et/ou le résultat de pronostic d'une insuffisance cardiaque sans aucun malaise chez des patients de la classe I de NYHA selon la revendication 1, **caractérisé en ce que** le marqueur est le marqueur MR-proANP (AS 53-90 du NT-proANP).

3. Procédé pour le diagnostic in vitro, et/ou la stratification du risque, et/ou le résultat de pronostic d'une insuffisance cardiaque sans aucun malaise chez des patients de la classe I de NYHA selon les revendications 1 ou 2, **caractérisé en ce que** le patient a un indice de masse corporelle d'au moins 30 kg/m².

4. Procédé pour le diagnostic in vitro, et/ou la stratification du risque, et/ou le résultat de pronostic d'une insuffisance cardiaque sans aucun malaise chez des patients de la classe I de NYHA selon l'une des revendications 1 à 3, pour permettre des prises de décision cliniques, particulièrement le traitement continu et la thérapie au moyen d'une médication, particulièrement en médecine intensive ou en médecine d'urgence et pour l'hospitalisation de patients.

5. Procédé pour le diagnostic in vitro, et/ou la stratification du risque, et/ou le résultat de pronostic d'une insuffisance cardiaque sans aucun malaise chez des patients de la classe I de NYHA selon l'une des revendications 1 à 4, pour la détection précoce par un diagnostic différentiel et la détection, l'évaluation de la sévérité, et pour la thérapie accompagnant l'évaluation du développement.

6. Procédé selon l'une des revendications précédentes, **caractérisé en ce qu'**une détermination d'au moins un autre marqueur, choisi dans le groupe des marqueurs inflammatoires, des marqueurs cardiovasculaires, ou des marqueurs ischémiques, est réalisée de manière complémentaire sur un patient devant être examiné.

7. Procédé selon l'une des revendications précédentes, **caractérisé en ce que** le marqueur inflammatoire est choisi parmi au moins un marqueur du groupe constitué de la protéine C-réactive (CRP), de cytokines, telles que la TNF-alpha, d'interleukines, telles que l'IL-6, de pro calcitonine (1-116, 3-116), et de molécules d'adhérence, telles que la VCAM ou l'ICAM.

8. Procédé selon l'une des revendications précédentes, **caractérisé en ce que** le marqueur cardiovasculaire est choisi parmi au moins un marqueur du groupe constitué de la kinésie de la créatine, de la myéloperoxydase, de la copeptine, de la myoglobine, de la proponine cardiaque, de la CRP, et de (pro)hormones régulant la circulation, telles que le peptide libérant la pro-gastrine (proGRP), la pro-endothéline (proEnd), la pro-leptine, le pro-neuropeptide-Y, la pro-somatostatine, le pro-neuropeptide YY, la pro-opiomélanocortine, la pro-adrénomédulline (proADM), la copeptine, un une séquence partielle de ceux-ci.

9. Procédé selon l'une des revendications précédentes, **caractérisé en ce que** le marqueur ischémique est choisi parmi au moins un marqueur du groupe des troponines I et T, CK-MB.

10. Procédé selon l'une des revendications précédentes, **caractérisé en ce que** des déterminations parallèles ou simultanées des marqueurs sont réalisées.

11. Procédé selon l'une des revendications précédentes, **caractérisé en ce que** les déterminations sont réalisées sur au moins un échantillon du patient.

12. Procédé selon l'une des revendications précédentes, **caractérisé en ce que** les déterminations sont réalisées sur un analyseur automatique, particulièrement au moyen d'un Kryptor.

13. Procédé selon l'une des revendications précédentes, **caractérisé en ce que** les déterminations sont réalisées au moyen d'un test rapide, particulièrement dans des déterminations de paramètres individuels ou multiples.

14. Utilisation des marqueurs proANP ou NT-proANP, ou MR-proANP (AS 53-90 du NT-proANP) parallèlement à une détermination des marqueurs proBNP, NT-proBNP, et/ou BNP d'un patient qui doit être examiné pour le diagnostic in vitro, et/ou la stratification du risque, et/ou le résultat de pronostic d'une insuffisance cardiaque sans aucun malaise chez des patients de la classe 1 de NYHA.


### SEQUENCE LISTING

<110> Brahms Aktiengesellschaft
<120> Diagnose und Risikostratifizierung von Herzinsuffizienz mittels natriuretischen Peptiden für NYHA I Patienten
<130> BRAHMS 34WO
<140> PCT/DE2008/
   <141> 2008-03-03
<150> DE10 2007 010 834.8
   <151> 2007-03-03
<160> 2
<170> PatentIn version 3.3
<210> 1
   <211> 126
   <212> PRT
   <213> Homo sapiens
<400> 1
   Asn Pro Met Tyr Asn Ala Val Ser Asn Ala Asp Leu Met Asp Phe Lys
   1               5                   10                  15
   Asn Leu Leu Asp His Leu Glu Glu Lys Met Pro Leu Glu Asp Glu Val
               20                  25                  30
   Val Pro Pro Gln Val Leu Ser Glu Pro Asn Glu Glu Ala Gly Ala Ala
           35                  40                  45
   Leu Ser Pro Leu Pro Glu Val Pro Pro Trp Thr Gly Glu Val Ser Pro
       50                  55                  60
   Ala Gln Arg Asp Gly Gly Ala Leu Gly Arg Gly Pro Trp Asp Ser Ser
   65                  70                  75                  80
   Asp Arg Ser Ala Leu Leu Lys Ser Lys Leu Arg Ala Leu Leu Thr Ala
                   85                  90                  95
   Pro Arg Ser Leu Arg Arg Ser Ser Cys Phe Gly Gly Arg Met Asp Arg
               100                 105                 110
   Ile Gly Ala Gln Ser Gly Leu Gly Cys Asn Ser Phe Arg Tyr
           115                 120                 125
<210> 2
   <211> 108
   <212> PRT
   <213> Homo sapiens
<400> 2
   His Pro Leu Gly Ser Pro Gly Ser Ala Ser Asp Leu Glu Thr Ser Gly
   1               5                   10                  15
   Leu Gln Glu Gln Arg Asn His Leu Gln Gly Lys Leu Ser Glu Leu Gln
               20                  25                  30
   Val Glu Gln Thr Ser Leu Glu Pro Leu Gln Glu Ser Pro Arg Pro Thr
           35                  40                  45
   Gly Val Trp Lys Ser Arg Glu Val Ala Thr Glu Gly Ile Arg Gly His
       50                  55                  60
   Arg Lys Met Val Leu Tyr Thr Leu Arg Ala Pro Arg Ser Pro Lys Met
   65                  70                  75                  80
   Val Gln Gly Ser Gly Cys Phe Gly Arg Lys Met Asp Arg Ile Ser Ser
                   85                  90                  95
   Ser Ser Gly Leu Gly Cys Lys Val Leu Arg Arg His
               100                 105